(19) Europäisches Patentamt
European Patent Office
Office européen des brevets

(11) **EP 2 148 686 B1**

(12) **FASCICULE DE BREVET EUROPEEN**

(45) Date de publication et mention de la délivrance du brevet:
**21.05.2014 Bulletin 2014/21**

(21) Numéro de dépôt: **08805836.7**

(22) Date de dépôt: **23.05.2008**

(51) Int Cl.:
*A61K 31/7028* (2006.01)  *A61K 31/795* (2006.01)
*A61L 15/44* (2006.01)  *A61P 17/02* (2006.01)
*A61L 15/22* (2006.01)  *A61F 13/00* (2006.01)
*A61K 8/81* (2006.01)  *A61Q 19/00* (2006.01)

(86) Numéro de dépôt international:
**PCT/FR2008/050892**

(87) Numéro de publication internationale:
**WO 2008/149035 (11.12.2008 Gazette 2008/50)**

(54) **COPOLYMÈRE UTILISÉ POUR FAVORISER LA PROLIFÉRATION DES FIBROBLASTES**

COPOLYMER ZUR VERBESSERUNG DER VERMEHRUNG VON FIBROBLASTEN

COPOLYMER FOR ENHANCING THE PROLIFERATION OF FIBROBLASTS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **25.05.2007 FR 0755271**

(43) Date de publication de la demande:
**03.02.2010 Bulletin 2010/05**

(73) Titulaire: **Laboratoires Urgo**
**21300 Chenove (FR)**

(72) Inventeur: **LAURENSOU, Christelle**
**F-21000 Dijon (FR)**

(74) Mandataire: **Hubert, Philippe et al**
**Cabinet Beau de Loménie**
**158, rue de l'Université**
**75340 Paris Cedex 07 (FR)**

(56) Documents cités:
**JP-A- 2006 342 098**

• **WEST J L: "Polymers for the management of wound healing" TRENDS IN POLYMER SCIENCE, ELSEVIER SCIENCE PUBLISHERS B.V. AMSTERDAM, NL, vol. 4, no. 7, juillet 1996 (1996-07), pages 206-207, XP004049268 ISSN: 0966-4793**

**Description**

La présente invention a essentiellement pour objet une nouvelle utilisation d'un composé qui est un copolymère d'un sel de l'acide 2-méthyl-2-[(1-oxo-2-propènyl)amino]-1-propanesulfonique et de l'ester 2-hydroxyéthyle de l'acide propénoïque.

Plus précisément, la présente invention concerne l'utilisation de ce composé comme agent favorisant et/ou accélérant la prolifération et/ou la différenciation des fibroblastes notamment pour la préparation d'une composition destinée à favoriser la cicatrisation ou encore pour la préparation d'une composition destinée à être utilisée ex vivo pour la génération de cellules pour des greffes de peau.

L'invention concerne également un pansement pour le traitement des plaies contenant ce composé, seul ou associé à d'autres substances actives.

On sait que les plaies ouvertes, qui comprennent les brûlures, les ulcères, ulcères neuropathiques, ulcères veineux ou artériels, les ulcères diabétiques, les escarres, les ampoules, les plaies exsudatives, les lésions dermoépidermiques superficielles, chroniques ou aigües, représentent l'immense majorité des plaies.

Un des premiers objectifs dans le traitement de la plaie, quelle qu'en soit la nature ou l'origine, est de fermer cette plaie.

On sait par ailleurs que la cicatrisation d'une plaie est un phénomène biologique naturel, les tissus humains et animaux étant capables de réparer des lésions localisées par des processus de réparation et de régénération qui leurs sont propres.

D'une façon générale, la cicatrisation d'une plaie se déroule en trois phases : la phase de détersion, la phase de bourgeonnement et la phase d'épithélialisation, chacune de ces phases étant caractérisée par des activités cellulaires spécifiques qui font progresser le processus de réparation selon des séquences chronologiques précises.

La phase de détersion est caractérisée par l'apparition de phénomènes inflammatoires précoces. Immédiatement après le traumatisme débutent des sécrétions provenant des vaisseaux sanguins et lymphatiques. La coagulation est induite par activation de la thrombokinase qui est libérée avec la formation corrélative de fibrine. Après 10 minutes environ, débute l'exsudation qui va assurer la défense contre l'infection et la détersion de la plaie. Environ 4 jours après la blessure commence la phase proliférative. L'organisme commence à combler la perte de substance par un nouveau tissu. Dans ce but les fibroblastes produisent en premier lieu des mucopolysaccharides qui serviront de matrice à l'élaboration des fibres collagènes du tissu conjonctif. Entre le 6ème et le 10ème jour en moyenne vient la phase de différenciation. La maturation des fibres collagènes commence. La plaie se rétracte sous l'influence de cellules particulières, les myofibroblastes. En s'appauvrissant progressivement en eau et en contenant de moins en moins de vaisseaux, le tissu de granulation devient plus ferme. Il se transforme en tissu cicatriciel qui, à son tour, favorisera la rétraction cicatricielle.

C'est au cours de la seconde phase de la cicatrisation, appelée phase de bourgeonnement que les fibroblastes vont jouer un rôle important. La phase de bourgeonnement dure environ 3 semaines et, lors de la cicatrisation normale, l'épithélialisation se prépare également dès ce moment. Des fibroblastes se multiplient au sein de la plaie, ainsi que des cellules précurseurs des kératinocytes, à partir des berges de la plaie, des follicules pileux et des glandes sudoripares. Après 3 jours, les fibroblastes produisent du collagène, dont les fibres s'orientent selon les forces auxquelles elles sont soumises. Leur prolifération est régie par un certain nombre de facteurs. L'arrêt de cette prolifération se fait en règle générale lorsque le tissu de granulation a comblé la perte de substance et lorsque la prolifération fibroblastique est remontée au niveau des berges.

Le fibroblaste est l'un des agents majeurs dans le processus de cicatrisation. En effet, vers le 6ème jour, plus de la moitié des fibroblastes présents dans la plaie se transforment en myofibroblastes. Ces cellules se caractérisent par la présence, au sein de leur cytoplasme, de myofibrilles contractiles qui provoquent la contraction de la plaie, et par conséquent la diminution de sa surface et l'accélération de sa fermeture.

Ces cellules ont donc un rôle essentiel dans la contraction de la plaie, phénomène majeur de fermeture spontanée des plaies cavitaires. Plus de 40% des myofibroblastes (contenant, une protéine contractile, l'alpha smooth muscle actine) sont présents dans le tissu de bourgeonnement. Lorsque la plaie est cicatrisée, ces cellules meurent sans que le signal déclenchant leur disparition soit parfaitement connu.

Le fibroblaste est également la cellule clef de la phase proliférative. Le fibroblaste va sécréter du collagène type III, puis ultérieurement du collagène type I, de l'héparane sulfate, constituants fondamentaux de la matrice extracellulaire du derme, mais aussi de l'acide hyaluronique, de la chondroïtine sulfate, de la fibronectine et une collagénase, ce qui va conduire à la fermeture de la plaie.

Le processus de cicatrisation, même pour de petites plaies ou brèches, peut durer pendant une période de quelques heures, quelques jours ou quelques semaines, voire même plus comme dans certaines circonstances, telles que l'ulcération où la plaie peut persister pour des périodes beaucoup plus longues, i.e., des mois ou des années.

Durant cette période de cicatrisation, qu'elle soit de courte ou de longue durée, la plaie peut être sujette à tout type d'invasions (organismes pathogènes ou substances étrangères) jusqu'à la régénération d'un nouveau tissu qui ferme complètement la brèche.

Pour éviter toute infection, la plaie est habituellement traitée afin d'enlever tout contaminant (poussières, débris...) susceptible d'introduire une matière pathogène au niveau de la zone lésée. On effectue ensuite un débridement des tissus au niveau de cette zone et une aseptisation. Dans certains cas, on réalise en outre un certain nombre de points de suture afin de faciliter la fermeture de la plaie. Une fois ces étapes réalisées, la plaie est maintenue dans un environnement favorable à la cicatrisation. Pour ce faire, différents types de pansements sont utilisés afin d'éviter l'intrusion de pathogènes et/ou leur prolifération.

Il est donc important, lorsque l'on traite une plaie, de favoriser le phénomène de fermeture de la plaie afin d'éviter, par exemple, l'invasion de la plaie par des microorganismes ou des substances étrangères, et par conséquent l'infection de la plaie. De ce fait, il est nécessaire de disposer de moyens permettant d'induire ou de favoriser le processus de cicatrisation de la plaie.

Afin d'induire ou d'accélérer la cicatrisation de la plaie, on peut administrer un certain nombre d'actifs. Ces actifs peuvent agir à différents niveaux et aux différentes phases de la cicatrisation. Ils diffèrent en fonction du stade et du type de plaie à traiter. Ces actifs peuvent être, par exemple, des facteurs de croissance des fibroblastes et/ou des kératinocytes (tels que le Basic Fibroblast Growth factor) ou des pseudo facteurs de croissance (tels que le mannose 6 phosphate), des glycosaminoglycanes (tels que l'acide hyaluronique, le collagène...), des hormones (telles que l'oestradiol, la DHEA...), des polysaccharides (tels que le dextrane)...

Dans l'article « Polymers for the management of wound healing » de Jennifer L. West publié dans TRIP vol .4, n°7, juillet 1996, l'utilisation de polymères tels que le polyacide glycolique, le collagène réticulé par le glycosaminoglycane, pour la cicatrisation des plaies est envisagée.

Il a été découvert, de façon tout à fait surprenante et inattendue, qu'il était également possible de favoriser et/ou d'accélérer la prolifération et/ou la différenciation des fibroblastes et par conséquent de favoriser la cicatrisation au moyen d'un composé qui est un copolymère d'un sel de l'acide 2-méthyl-2-[(1-oxo-2-propènyl)amino]-1-propanesulfonique et de l'ester 2-hydroxyéthyle de l'acide propénoïque.

De préférence, le sel de l'acide 2-méthyl-2-[(1-oxo-2-propènyl)-amino]-1-propane-sulfonique précité est un sel de sodium.

Ce copolymère est un produit connu en tant que tel notamment dans le domaine cosmétique en raison de ses propriétés émulsionnantes-stabilisantes et de son bon pouvoir épaississant (voir JP 2006 342098).

Un tel produit est, par exemple, commercialisé par la société SEPPIC sous la dénomination commerciale SEPINOV EMT 10®.

Ainsi, selon un premier aspect, la présente invention a pour objet l'utilisation d'un copolymère d'un sel de l'acide 2-méthyl-2-[(1-oxo-2-propènyl)amino]-1-propanesulfonique et de l'ester 2-hydroxyéthyle de l'acide propénoïque pour la préparation d'une composition destinée à favoriser et/ou à accélérer la prolifération des fibroblastes in vivo ou ex vivo, et en particulier pour la préparation d'une composition destinée à favoriser la cicatrisation.

Le copolymère précité peut être utilisé in vivo ou ex vivo.

Comme exemple d'application ex vivo on peut citer l'utilisation de ce composé pour la préparation d'un milieu de culture pour favoriser la prolifération des fibroblastes. Cette utilisation peut s'avérer utile pour les greffes de feuillets dermiques ou dermo-épidermiques autologues. En effet, dans ce type de greffe on utilise des techniques de culture cellulaire afin d'obtenir une surface de peau suffisante à partir d'un petit échantillon prélevé chez le patient lui-même. L'utilisation du copolymère selon la présente invention permet d'accélérer la prolifération des fibroblastes et peut donc s'avérer utile dans ce type de traitement.

Dans le cadre de son application in vivo, le copolymère précité peut être utilisé pour la préparation d'une composition qui permettra son application directement au niveau de la plaie ou de son environnement, ou encore au niveau des muqueuses, avantageusement par voie topique. De manière préférentielle, ce copolymère est intégré à un pansement.

D'une façon générale, le copolymère précité peut être utilisé seul ou en combinaison avec d'autres substances actives permettant d'induire ou d'accélérer la cicatrisation ou pouvant avoir un rôle favorable dans le traitement de la plaie, telle que, par exemple, les antimicrobiens, les anti-douleurs. Une telle association d'actifs permet un traitement médical combiné de la plaie. Parmi les substances actives susceptibles d'être utilisées dans le cadre de l'invention, on peut citer, à titre d'exemples, des agents bactéricides ou bactériostatiques (chloramine, chlorhexidine, sels d'argent, sels de zinc, metronidazole, néomycine...), des agents favorisant la cicatrisation (hormones, peptides...), des enzymes favorisant la détersion de la plaie (pepsine, trypsine...), des inhibiteurs de protéase ou métalloprotéase, des agents anti-douleurs ou des anesthésiques locaux (lidocaïne, chinchocaïne) ou des agents anti-inflammatoires non stéroïdiens (ibuprofène, kétoprofène, fénoprofène, diclofénac).

De manière préférentielle, le copolymère précité sera utilisé en combinaison avec un saccharide sulfaté ou un sel ou complexe de saccharide sulfaté. Ce saccharide sulfaté peut être, par exemple, un sucrose octasulfate sous forme d'un complexe ou d'un sel avec un métal tel que Na, K, Ca, Mg, Ba, Al, Zn, Cu, Zr, Ti, Mn, ou Os, ou avec une base organique, telle qu'un acide aminé. Des résultats particulièrement intéressants ont été obtenus lorsque le copolymère précité est utilisé en combinaison avec le sucrose octasulfate de potassium qui est un principe actif connu pour son action dans le processus de cicatrisation.

Des études de prolifération in vitro ont permis de montrer de façon inattendue qu'un copolymère d'un sel de l'acide 2-méthyl-2-[(1-oxo-2-propènyl)amino]-1-propanesulfonique et de l'ester 2-hydroxyéthyle de l'acide propénoïque présente une activité sur la prolifération des fibroblastes à très faible dose. En effet, une telle activité a été constatée lorsque le copolymère est présent dans le milieu de culture à une concentration de 0,00064 mg/ml.

Il a également été montré que, de manière particulièrement avantageuse, le copolymère précité présente en outre une activité sur plusieurs jours. Des tests effectués in vivo ont permis de déceler une activité sur la prolifération des fibroblastes 72 heures après son incorporation au milieu de culture. Ceci est particulièrement avantageux lorsque le copolymère est incorporé à un pansement. En effet, un même pansement pourra être utilisé plusieurs jours tout en restant actif sans qu'il ne soit besoin de le changer.

D'une façon générale, le copolymère utilisé dans le cadre de la présente invention peut être intégré dans tout type de composition, comme en particulier une solution, une crème, un gel, une masse, notamment une masse élastomérique ou un pansement. Le copolymère peut être intégré à une compresse. Par compresse on entend tout type de matériau absorbant habituellement utilisé dans les pansements, tels que, par exemple, les matériaux textiles qui peuvent être des non-tissés absorbants (à base de viscose, par exemple) associés ou non à des non-tissés non absorbants (telles que des fibres de polyester). Il peut également s'agir de fibres super-absorbantes (telles que les Lanseal® Fibres commercialisées par Toyobo co, Ltd) ou de mousses en polyuréthane.

En particulier, ce copolymère peut être présent dans la masse constituant un pansement ou sur une couche séparée du pansement ou encore dans un revêtement couvrant la surface du pansement destinée à venir en contact avec la plaie afin de la traiter.

Par pansement on entend couvrir ici tout type de pansements connus et d'une façon préférée les pansements interfaces. De tels pansements sont commercialisés par exemple sous les dénominations commerciales Tulle Gras® (par SOLVAY PHARMA), Physiotulle® (par COLOPLAST) ou encore Urgotul® (par les Laboratoires URGO) et décrits dans le brevet EP 1143 895.

Ces pansements interfaces se présentent généralement sous forme d'une trame ou d'un filet enduit d'une masse habituellement une masse élastomérique. Ils peuvent également être constitués d'une masse sans trame ou filet, ayant la forme d'une plaque présentant ou non des trous traversants, en fonction du type de plaie sur lequel le pansement est appliqué (on utilisera préférentiellement une plaque présentant des trous traversants sur une plaie exsudative lorsque la masse n'a qu'un faible ou aucun pouvoir absorbant, les trous permettant ainsi l'évacuation des exsudats de la plaie).

La présente invention trouve également application pour la réalisation de pansements à base d'hydrogels ou d'hydrocolloïdes dans lesquels est incorporé le copolymère précité. Des pansements à base d'hydrocolloïdes connus sont par exemple commercialisés sous les dénominations Algoplaque® (par les Laboratoires Urgo), Duoderm® (par Convatec), Comfeel® (par Coloplast). De tels pansements sont décrits dans les demandes de brevet suivantes : FR 2 392 076, FR 2 495 473 et WO 98/10801, EP 264 299.

Le copolymère utilisé dans le cadre de la présente invention peut être incorporé à un élément absorbant telle qu'une compresse ou une mousse, par exemple en le déposant sur la surface destinée à venir au contact avec la plaie, comme décrit dans la demande de brevet WO 2006/007844.

La présente invention trouve également application pour la réalisation de pansements interfaces complexés à une couche absorbante telle qu'une mousse ou une compresse, ou d'une masse hydrocolloïde complexée à une mousse absorbante. De tels pansements sont connus et commercialisés par exemple sous les dénominations commerciales UrgotulDuo® et Cellosorb® (par les Laboratoires Urgo). Dans de tels pansements, le copolymère précité peut être incorporé à la masse et/ou à la couche absorbante.

Les pansements interfaces utilisés de manière préférentielle dans le cadre de la présente invention sont constitués d'une masse élastomérique, c'est à dire constituée à partir d'un ou plusieurs élastomères choisis parmi les copolymères séquencés poly(styrène-oléfine-styrène) et un ou plusieurs composés choisis parmi les plastifiants, les résines tackifiantes et, si nécessaire, les antioxydants.

De telles masses élastomériques sont bien connues de l'homme du métier et sont décrites par exemple dans "Advances in Pressure Sensitive Adhesive Technology" édité par Donatas Satas en avril 1995 dans le chapitre 7 "Wound dressings" pages 158 à 171.

Lorsqu'il est intégré à un pansement, tel qu'un pansement interface, le copolymère précité peut être utilisé en une concentration de 1 à 5 % en poids rapportée au poids de la masse élastomérique. Il a été montré qu'une telle concentration est suffisante pour activer la prolifération fibroblastique et par conséquent favoriser la cicatrisation.

D'une façon générale, le copolymère précité sera présent dans un pansement en une quantité pouvant être comprise entre 0,1 et 20% en poids rapportée au poids de la masse dans laquelle il est incorporé, et de préférence comprise entre 1 et 5% en poids.

Selon un deuxième aspect, la présente invention vise à couvrir les pansements décrits précédemment qui incorporent un copolymère d'un sel de l'acide 2-méthyl-2-[(1-oxo-2-propènyl)amino]-1-propanesulfonique et de l'ester 2-hydroxyéthyle de l'acide propénoïque.

Pour mettre en évidence l'activité du copolymère précité sur les fibroblastes, une étude in vitro a été réalisée, mettant en oeuvre deux types d'expériences :

1. Le copolymère seul ou en combinaison avec une substance active (en l'occurrence le sucrose octasulfate de potassium) a été ajouté directement au milieu de culture des fibroblastes à différentes concentrations.

Dans ce cadre, différentes dilutions du copolymère, seul ou en combinaison avec le sucrose octasulfate de potassium, ont ainsi été réalisées et ajoutées au milieu de culture.

Des dilutions du copolymère Sepinov EMT 10 à 0,011 mg/mL et de sucrose octasulfate de potassium à 0,00064 mg/mL ont ainsi été testées dans un milieu de culture comprenant des fibroblastes.

2. Le copolymère seul ou en combinaison avec une substance active (en l'occurrence le sucrose octasulfate de potassium) a été intégré à la matrice d'un pansement interface, puis le pansement a été appliqué sur les puits de culture contenant les fibroblastes.

Dans ce cadre, différents pansements interfaces contenant le copolymère à différentes concentrations, en combinaison ou non avec du sucrose octasulfate de potassium ont été fabriqués en suivant le procédé décrit ci-après. Un pansement interface ne contenant aucun de ces deux actifs a également été fabriqué et testé, à titre comparatif.

Procédé de fabrication des pansements testés

a. Fabrication des masses élastomériques

Diverses masses élastomériques (exemples 1 à 5) ont été élaborées à l'aide des constituants suivants, dans les proportions en poids mentionnées dans le tableau 1 :

- Elastomère : copolymère séquencé de poly(styrène-éthylène-butylène-styrène) (en abrégé SEBS) ; Kraton G 1654 et G 1651 commercialisés par la société KRATON
- Plastifiant : huile minérale : Ondina 917 commercialisée par la société SHELL
- Antioxydant : IRGANOX 1010 commercialisé par la société CIBA SPECIALTY CHEMICALS
- Corps gras : vaseline : Vaseline Codex A commercialisé par la société AIGLON
- Hydrocolloïde : carboxyméthylcellulose sodique : CMC Blanose 7H4XF commercialisé par la société HERCULES
- Substance actives additionnelle : sucrose octasulfate de potassium : commercialisé par la société EUTICALS
- Copolymère d'un sel de l'acide 2-méthyl-2-[(1-oxo-2-propènyl)amino]-1-propanesulfonique et de l'ester 2-hydroxyéthyle de l'acide propénoïque : SEPINOV EMT 10 commercialisé par la société SEPPIC.

La masse à base d'élastomère a été préparée par malaxage dans un malaxeur à bras Z à une température de consigne de 155°C :

1. Les élastomères triblocs styrène-éthylènebutylène-styrène sont mélangés à la moitié de l'huile minérale et à l'antioxydant.
2. A la 30ème minute, la vaseline est ajoutée au mélange.
3. A la 40ème minute, le reste d'huile minérale est ajoutée.
4. A la 55eme minute, la carboxyméthylcellulose sodique, le cas échéant le ou les actif(s) sont ajoutés au mélange.

La vidange du malaxeur est effectuée à la 70ème minute.

b. Fabrication des pansements

Des pansements interfaces constitués d'une trame enrobée d'une masse élastomérique ont été élaborés à l'aide des masses élastomériques précitées.

Plus précisément, on a utilisé ici une trame formée d'une marquisette thermofixée en fils de polyester (polyéthylène téréphtalate), 33 décitex en chaîne et en trame, présentant des mailles carrées dont l'ouverture est d'environ 0,8 à 1 mm$^2$ (trame 555 commercialisée par la société MDB TEXINOV).

Cette trame a été enrobée d'une couche de masse élastomérique fondue à 135-145°C, puis l'excédent a été éliminé par passage entre deux cylindres fixes dont l'écart est de 200 $\mu$m. La bande ainsi obtenue a été découpée puis complexée avec un film protecteur en polyester d'épaisseur de 23 $\mu$m sur chacun de ses faces formant ainsi des pansements individuels conditionnés dans des pochettes étanches et stérilisés sous rayonnement $\beta$ à 25 kGy.

Tableau 1

| Constituants | Exemple 1 | Exemple 2 | Exemple 3 | Exemple 4 | Exemple 5 |
|---|---|---|---|---|---|
| Huile minérale (Ondina 917) | 75 | 74 | 70 | 62,38 | 69,9 |
| S-EB-S (Kraton G 1651) | 4,9 | 4,9 | 4,9 | | |
| S-EB-S (Kraton G 1654) | | | | 6 | 6 |
| Antioxydant (Irganox 1010) | 0,1 | 0,1 | 0,1 | 0,12 | 0,1 |
| Vaseline (Vaseline Codex A) | 5 | 5 | 5 | 5 | 5 |
| Carboxyméthylcellulose (CMC Blanose 7H4XF) | 15 | 15 | 15 | 14 | 14 |
| SEPINOV EMT 10 | | 1 | 5 | 5 | 5 |
| Sucrose octasulfate de potassium | | | | 7,5 | |

L'effet du copolymère de l'acide 2-méthyl-2-[(1-oxo-2-propènyl)amino]-1-propanesulfonique et de l'ester de 2-hydroxyéthyle de l'acide propénoïque sur la prolifération des fibroblastes a été démontré selon le protocole suivant.

**Mise en évidence de l'activité du copolymère précité sur la prolifération des fibroblastes :**

Cellules utilisées :

Type : pool de fibroblastes dermiques humains normaux (NHDF) R9PF2 Culture : 37°C, 5% CO2
Milieu de culture :

DMEM (Dulbecco's Modified Eagle Medium, Invitrogen 21969035)
L-glutamine 2mM (Invitrogen 25030024)
Pénicilline 50 UI/mL (Invitrogen 15070063)
Sérum de veau foetal 10% (v/v, Invitrogen 10270098)

Produits testés :

Des pansements à la dimension des puits préparés comme décrits précédemment à l'aide des masses élastomériques des exemples 1 à 5, ainsi que des dilutions du copolymère Sepinov EMT 10 à 0,011 mg/mL et de sucrose octasulfate de potassium à 0,00064 mg/mL ont été testés.

Effets sur la prolifération :

Les fibroblastes ont été ensemencés en plaques 12 puits à faible densité (60 % de confluence), puis les cellules ont été traitées par les dilutions ou des morceaux de pansements ont été appliqués sur ces plaques et maintenus à l'aide d'un bouchon (appelé « test »). Un contrôle sans pansement, sans dilution avec bouchon a été réalisé (appelé « témoin »).
Les cellules ont ensuite été incubées pendant 24 heures, 48 heures et 72 heures. Pour chaque temps d'incubation, la thymidine tritiée ([méthyl-3H]-thymidine, Amersham TRK 686 2.5$\mu$Ci/mL final) a été ajoutée pendant les 24 dernières heures d'incubation, puis l'ADN des cellules des tapis cellulaires a été extrait, purifié et la radioactivité incorporée dans l'ADN a été comptée à l'aide d'un compteur à scintillation.
les résultats obtenus sont exprimés en coups par minute (cpm), puis en pourcentage par rapport au témoin selon la formule suivante :

$$\%_{témoin} = (cpm_{test} / cpm_{témoin}) \times 100$$

dans laquelle :

cpm$_{test}$ : nombre de coups par minute obtenus avec le test
cpm$_{témoin}$ : nombre de coups par minute obtenus avec le témoin

Toutes les conditions ont été réalisées en triplicata.
Les résultats obtenus figurent dans les tableaux 2 à 4 ci-dessous.

Tableau 2: Résultats de l'étude de prolifération des NHDF après incorporation de la thymidine tritiée à 24 heures

| Traitement | $\%_{témoin}$ |
|---|---|
| Témoin | 100 |
| Pansement selon l'exemple 1 | 130 |
| Pansement selon l'exemple 2 | 196 |

Tableau 3 Résultats de l'étude de prolifération des NHDF après incorporation de la thymidine tritiée à 48 heures

| Traitement | %témoin |
|---|---|
| Témoin | 100 |
| Pansement de l'exemple 1 | 181 |
| Pansement de l'exemple 2 | 260 |
| Pansement de l'exemple 3 | 506 |
| Pansement de l'exemple 4 | 456 |
| Pansement de l'exemple 5 | 243 |
| Sucrose octasulfate de potassium à 0,011 mq/mL | 106 |
| Sepinov EMT 10 à 0,00064 mq/mL | 125 |
| Sucrose octasulfate de potassium à 0,011 mg/mL et Sepinov EMT 10 à 0,00064 mg/mL | 152 |

Tableau 4: résultat de l'étude de prolifération des NHDF après incorporation de la thymidine tritiée à 72 heures

| Traitement | %témoin |
|---|---|
| Témoin | 100 |
| Pansement selon l'exemple 1 | 150 |
| Pansement selon l'exemple 2 | 120 |
| Pansement selon l'exemple 3 | 493 |
| Pansement selon l'exemple 4 | 292 |
| Sepinov EMT 10 à 0,00064 mg/mL | 147 |

Ces expériences in vitro ont ainsi permis de mettre en évidence le rôle du copolymère SEPINOV EMT 10 sur la prolifération des fibroblastes ainsi que l'activité combinée de ce copolymère et du sucrose octasulfate de potassium sur ces mêmes cellules.

L'effet obtenu est particulièrement intéressant lorsque le copolymère SEPINOV EMT 10 est intégré dans une masse élastomérique à une concentration de 5% de la masse totale, et ce après 48h et 72h de mise en contact avec le milieu de culture cellulaire.

Le copolymère utilisé seul en dilution dans le milieu de culture cellulaire à une concentration très faible (0,00064 mg/mL) a également favorisé la prolifération des fibroblastes de manière significative.

On a également pu constater que, de manière surprenante, lorsque le copolymère SEPINOV EMT 10 est utilisé en combinaison avec le sucrose octasulfate de potassium, il se produit un effet de synergie sur la prolifération des fibroblastes. Cette synergie a été observée en particulier lorsque le sucrose octasulfate de potassium est ajouté au milieu de culture à une concentration de 0,011 mg/mL et le copolymère Sepinov EMT 10 à une concentration de 0,00064 mg/mL.

Le copolymère utilisé selon la présente invention est donc particulièrement avantageux pour le traitement des plaies, et en particulier pour favoriser la cicatrisation notamment lorsqu'il est incorporé à un pansement.

**Revendications**

1. Utilisation d'un copolymère d'un sel de l'acide 2-méthyl-2-[(1-oxo-2-propènyl)amino]-1-propanesulfonique et de l'ester 2-hydroxyéthyle de l'acide propénoïque pour la préparation d'une composition destinée à favoriser et/ou à accélérer la prolifération des fibroblastes in vivo.

2. Utilisation d'un copolymère d'un sel de l'acide 2-méthyl-2-[(1-oxo-2-propènyl)amino]-1-propanesulfonique et de l'ester 2-hydroxyéthyle de l'acide propénoïque pour favoriser et/ou à accélérer la prolifération des fibroblastes ex vivo.

3. Utilisation selon la revendication 1 pour la préparation d'une composition destinée à favoriser la cicatrisation.

4. Utilisation selon l'une des revendications 1 à 3, **caractérisée en ce que** le copolymère précité est associé à une ou plusieurs substances actives.

5. Utilisation selon l'une des revendications 1 à 4, **caractérisée en ce que** le copolymère précité est associé à un saccharide sulfaté, de préférence le sucrose octasulfate de potassium.

6. Utilisation selon l'une des revendications 1 à 5, **caractérisée en ce que** le sel de l'acide 2-méthyl-2-[(1-oxo-2-propènyl)amino]-1-propanesulfonique précité est un sel de sodium.

7. Pansement pour le traitement de la plaie, **caractérisé en ce qu'**il contient un copolymère d'un sel de l'acide 2-méthyl-2-[(1-oxo-2-propènyl)aminol-1-propanesulfonique et de l'ester 2-hydroxyéthyle de l'acide propénoïque.

8. Pansement pour le traitement selon la revendication 7 dans lequel le copolymère précité est associé à une ou plusieurs substances actives.

9. Pansement pour le traitement selon la revendication 8 dans lequel la substance active précitée est un saccharide sulfaté, de préférence le sucrose octasulfate de potassium.

10. Pansement pour le traitement selon l'une des revendications 7 à 9 dans lequel le copolymère précité est présent dans l'élément absorbant constitutif du pansement.

11. Pansement pour le traitement selon l'une des revendications 7 à 10 dans lequel le copolymère précité est présent dans une masse, de préférence une masse élastomérique, constitutive dudit pansement.

12. Pansement pour le traitement selon la revendication 11 dans lequel le copolymère précité est présent en une quantité comprise entre 0,1 et 20% en poids, et de préférence comprise entre 1 et 5% en poids, rapportée au poids de la masse dans laquelle il est incorporé.

13. Pansement pour le traitement selon l'une des revendications 7 à 12, **caractérisé en ce que** le sel de l'acide 2-méthyl-2-[(1-oxo-2-propènyl)amino]-1-propane-sulfonique précité est un sel de sodium.

**Patentansprüche**

1. Verwendung eines Copolymers aus einem Salz der 2-Methyl-2-[(1-oxo-2-propenyl)amino]-1-propansulfonsäure und dem Propensäure-2-Hydroxyethylester für die Zubereitung einer Zusammensetzung, welche dazu bestimmt ist, die Proliferation von Fibroblasten in vivo zu begünstigen und/oder zu beschleunigen.

2. Verwendung eines Copolymers aus einem Salz der 2-Methyl-2-[(1-oxo-2-propenyl)amino]-1-propansulfonsäure und dem Propensäure-2-Hydroxyethylester zum Begünstigen und/oder zum Beschleunigen der Proliferation von Fibroblasten ex vivo.

3. Verwendung nach Anspruch 1 für die Zubereitung einer Zusammensetzung, welche dazu bestimmt ist, die Wundheilung zu fördern.

**4.** Verwendung nach einem der Ansprüche 1 bis 3, **dadurch gekennzeichnet, dass** das vorgenannte Copolymer mit einem oder mehreren Wirkstoffen kombiniert ist.

**5.** Verwendung nach einem der Ansprüche 1 bis 4, **dadurch gekennzeichnet, dass** das vorgenannte Copolymer mit einem sulfatierten Saccharid, vorzugsweise Kalium Sucrose Octasulfat kombiniert ist.

**6.** Verwendung nach einem der Ansprüche 1 bis 5, **dadurch gekennzeichnet, dass** das vorgenannte Salz der 2-Methyl-2-[(1-oxo-2-propenyl)amino]-1-propansulfonsäure ein Natriumsalz ist.

**7.** Verband für die Wundbehandlung, **dadurch gekennzeichnet, dass** er ein Copolymer aus einem Salz der 2-Methyl-2-[(1-oxo-2-propenyl)amino]-1-propansulfonsäure und dem Propensäure-2-Hydroxyethylester enthält.

**8.** Verband für die Behandlung nach Anspruch 7, wobei das vorgenannte Copolymer mit einem oder mehreren Wirkstoffen kombiniert ist.

**9.** Verband für die Behandlung nach Anspruch 8, wobei der vorgenannte Wirkstoff ein sulfatiertes Saccharid, vorzugsweise Kalium Sucrose Octasulfat ist.

**10.** Verband für die Behandlung nach einem der Ansprüche 7 bis 9, wobei das vorgenannte Copolymer in dem den Verband bildenden absorbierenden Element vorhanden ist.

**11.** Verband für die Behandlung nach einem der Ansprüche 7 bis 10, wobei das vorgenannte Copolymer in einer den Verband bildenden Masse, vorzugsweise einer Elastomermasse vorhanden ist.

**12.** Verband für die Behandlung nach Anspruch 11, wobei das vorgenannte Copolymer in einer Menge zwischen 0,1 und 20 Gew.-% und vorzugsweise zwischen 1 und 5 Gew.-%, bezogen auf das Gewicht der Masse, in die es eingebettet ist, vorhanden ist.

**13.** Verband für die Behandlung nach einem der Ansprüche 7 bis 12, **dadurch gekennzeichnet, dass** das vorgenannte Salz der 2-Methyl-2-[(1-oxo-2-propenyl)amino]-1-propansulfonsäure ein Natriumsalz ist.

**Claims**

**1.** The use of a copolymer of a salt of 2-methyl-2-[(1-oxo-2-propenyl)amino]-1-propanesulfonic acid and of 2-hydroxyethylpropenoate ester, for the preparation of a composition intended to promote and/or accelerate fibroblast proliferation in vivo.

**2.** The use of a copolymer of a salt of 2-methyl-2-[(1-oxo-2-propenyl)amino]-1-propanesulfonic acid and of 2-hydroxyethylpropenoate ester, for promoting and/or accelerating fibroblast proliferation in vivo or ex vivo.

**3.** The use as claimed in claim 1, for the preparation of a composition intended to promote healing.

**4.** The use as claimed in one of claims 1 to 3, **characterized in that** the abovementioned copolymer is combined with one or more active substances.

**5.** The use as claimed in one of claims 1 to 4, **characterized in that** the abovementioned copolymer is combined with a sulfated saccharide, preferably potassium sucrose octasulfate.

**6.** The use as claimed in one of claims 1 to 5, **characterized in that** the abovementioned salt of 2-methyl-2-[(1-oxo-2-propenyl)amino]-1-propane-sulfonic acid is a sodium salt.

**7.** A dressing for wound treatment, **characterized in that** it contains a copolymer of a salt of 2-methyl-2-[(1-oxo-2-propenyl)amino]-1-propanesulfonic acid and of 2-hydroxyethylpropenoate ester.

**8.** The dressing for the treatment as claimed in claim 7, in which the abovementioned copolymer is combined with one or more active substances.

9. The dressing for the treatment as claimed in claim 8, in which the abovementioned active substance is a sulfated saccharide, preferably potassium sucrose octasulfate.

10. The dressing for the treatment as claimed in one of claims 7 to 9, in which the abovementioned copolymer is present in the absorbent element constituting the dressing.

11. The dressing for the treatment as claimed in one of claims 7 to 10, in which the abovementioned copolymer is present in a mass, preferably an elastomeric mass, constituting said dressing.

12. The dressing for the treatment as claimed in claim 11, in which the abovementioned copolymer is present in an amount of between 0.1% and 20% by weight, and preferably between 1% and 5% by weight, relative to the weight of the mass into which it is incorporated.

13. The dressing for the treatment as claimed in one of claims 7 to 12, **characterized in that** the abovementioned 2-methyl-2-[(1-oxo-2-propenyl)amino]-1-propanesulfonic acid salt is a sodium salt.

## RÉFÉRENCES CITÉES DANS LA DESCRIPTION

**Documents brevets cités dans la description**

- JP 2006342098 A
- EP 1143895 A
- FR 2392076
- FR 2495473
- WO 9810801 A
- EP 264299 A
- WO 2006007844 A

**Littérature non-brevet citée dans la description**

- **DE JENNIFER L. WEST.** Polymers for the management of wound healing. *TRIP,* Juillet 1996, vol. 4 (7
- Advances in Pressure Sensitive Adhesive Technology. Wound dressings. Avril 1995, 158-171